# EUROPEAN PATENT APPLICATION

(11) **EP 3 925 624 A1**
(43) Date of publication of application: **22.12.2021**
(21) Application number: 20756575.5
(22) Date of filing: 06.02.2020
(51) Int. Cl.: A61K 45/00, A61P 25/16, A61P 43/00, C08G 65/333, C08G 69/40, A61K 9/127, A61K 47/60, A61K 31/198

(54) **LEVODOPA DERIVATIVE AND USE THEREOF**

(30) Priority: 12.02.2019 JP 2019022895
(71) Applicant: University of Tsukuba, Ibaraki 305-8577 (JP)
(72) Inventor: NAGASAKI, Yukio, Tsukuba-shi, Ibaraki 305-8577 (JP); SATO, Yuna, Tsukuba-shi, Ibaraki 305-8577 (JP); VONG, Binh Long, Tsukuba-shi, Ibaraki 305-8577 (JP); CHONPATHOMPIKUNLERT, Pennapa, Khlong Luang, Pathumthani, 12120 (TH); HUTAMEKALIN, Pilaiwanwadee, Songkhla, 90112 (TH); TAKAHASHI, Reita, Tsukuba-shi, Ibaraki 305-8577 (JP)
(74) Representative: Beckmann, Claus
(86) International application number: PCT/JP2020/004494
(87) International publication number: WO 2020/166473

(57) **Abstract**

[Object]

To provide a drug that can mitigate shortcomings, such as low retention in blood, associated with L-DOPA in treating Parkinson's disease.

[Solution]

Incorporation of L-DOPA into a carrier in a form of a block copolymer (PEG-b-poly(3,4-hydroxy-protected L-DOPA)) significantly improves the retention of the agent in the blood.

## Description

### Technical Field

The present application provides a block copolymer comprising a poly(levodopa derivative), more specifically a block copolymer comprising a segment of poly(ethylene glycol) or a (PEG), a segment of poly(levodopa derivative), and use of the block copolymer, and also even more specifically an application of the block copolymer directed to the treatment of Parkinson's disease.

### Background Art

A condition caused by insufficient production of dopamine, a neurotransmitter, due to the reduction of dopamine cells in the brain by gene mutation, and developing symptoms such as muscle rigidity and tremor is referred to as Parkinson's disease (which may be hereinafter abbreviated as "PD"). To this date, no radical treatment is performed for PD, and L-DOPA, a precursor of dopamine that can cross the blood-brain barrier (BBB), which is a mechanism restricting the exchange of substances between the blood and the tissue fluid of the brain, is used as a therapeutic drug for PD (Non-Patent Literature 1).

L-DOPA (3,4-dihydroxy-L-phenylalanine or levodopa) is extremely superior in terms of efficacy in the early stage of treatment, low incidence of side effects, and inexpensiveness, whereas a short half-life is the largest drawback. In the late stage of treatment, a dopamine retention ability of dopamine neuronal cells decreases with the progression of the primary disease, thus further narrowing the therapeutic range. This makes it difficult to adjust the dosage of L-DOPA because of its low retention in blood, leading to the occurrence of a wearing-off phenomenon, where symptoms repeatedly improve and worsen during a course of a day, or dyskinesia due to overdose (Non-Patent Literature document 2).

In order to overcome these problems, attempts have been made, and there are several reports on cases aimed at providing prodrugs of L-DOPA, for example, esters or peptides of L-DOPA including esterification or L-DOPA (Patent Document 1), or improving the retention of L-DOPA in blood using nanoparticles. As to the nanoparticle case, for example, nanoparticles carried with levodopa methyl ester on PLGA can extend the half-life of L-DOPA in the blood (Non-Patent Literature 3), and micelles carried with chitosan as the outer shell and carried with dopamine as the core can permiate into the brain with micelles since chitosan loosens the tight junction of the BBB (Non-Patent Literature 4). However, the weak bonding strength in the nanoparticles described above can cause unexpected leakage of L-DOPA. For the latter example, it is suggested that transfer of toxins or the like in blood into the brain may occur at the same time as the loosening of the tight junction.

Only a small number of examples have been reported to date on effective dopamine delivery systems aimed at the treatment of Parkinson's disease. One of the reasons for this scarcity of the reports is the difficulty in handling L-DOPA due to the high reactivity of its two hydroxyl groups. L-DOPA readily undergoes oxidative polymerization in the atmosphere, producing a melanin-like viscous black substance and thus is applied as a surface modifier. However, the polymerized substance has strong carbon-carbon bonds, which cannot be degraded in vivo, and thus the use of L-DOPA as a prodrug is problematic (Non-Patent Literature 5). On the other hand, there has been a reported example in which L-DOPA is polymerized by peptide bonds, which can be degraded by enzymes in vivo, and the polymerized L-DOPA is utilized as an adhesive for living bodies. However, side reactions due to the oxidative polymerization during the synthesis are not taken into account (Non-Patent Literature 6). Thus, there is also a method in which the two hydroxyl groups are protected with acetyl groups in advance so as to prevent the oxidative polymerization during the reaction. However, this method is hazardous due to the use of hydrogen chloride (Non-Patent Literature 7).

### Citation List

### Patent Literature

Patent Document 1: US 3,998,799 B

### Non-Patent Literature

Non-Patent Literature 1: Kanda, T. (2008). Folia Pharmacol. Jpn., 131, 275-280
Non-Patent Literature 2: Cenci, M. A. (2014). Frontiers in neurology, 5, 242
Non-Patent Literature 3: Yang, X. (2012). International journal of nanomedicine, 7, 2077
Non-Patent Literature 4: Trapani, A. (2011). International journal of pharmaceutics, 419(1-2), 296-307
Non-Patent Literature 5: Xi, Z. (2009). Journal of Membrane Science, 327(1-2), 244-253.
Non-Patent Literature 6: Lu, D. (2017). ACS Appl. Mater. Interfaces, 9, 16756-16766
Non-Patent Literature 7: Gyu, H, H. (2017). Chem. Commun., 50, 4351-4353

### Summary of Invention

### Technical Problem

As described above, various L-DOPA derivatives or prodrugs have been proposed, but the therapeutic drug levodopa currently used for Parkinson's disease has the drawback of low retention in blood, and thus long-term relief of characteristic symptoms of Parkinson's disease and suppression of levodopa-induced dyskinesia (LID) are required. In addition, there is still a need to overcome the drawbacks of nanoparticles in the art for dopamine delivery systems aimed at the treatment of Parkinson's disease, such as the unexpected leakage of L-dopa and the damage to the BBB.

A block copolymer (PEG-b-poly(3,4-hydroxy-protected L-DOPA)), in which L-DOPA was incorporated into its carrier, was synthesized, and the use of such a copolymer was found to significantly reduce or resolve the problems described above. Without being bound by theory, the copolymer self-assembles in aqueous media, and this is presumed to improve the retention of the copolymer in blood of warm-blooded animals. In addition, enzymes present in the animal body presumably eliminate the 3,4-hydroxy protecting groups slowly and cleave the peptide bonds, releasing L-DOPA in the state of monomer, and this is presumed to allow L-DOPA to exert the intrinsic action. In the 3,4-hydroxy protected L-DOPA, the two hydroxyl groups, the main cause of the oxidative polymerization of L-DOPA are protected. This can suppress the polymerization and also hydrophobize the poly(L-DOPA), thus enabling the poly(L-DOPA) to readily self-assemble in an aqueous medium (aqueous solution or saline that may be buffered) to form a particle of several tens of nanometers in size. The self-assembled nanoparticles thus formed gradually degrade within the body of warm-blooded animals including humans and contribute to exerting a strong effect on Parkinson's disease.

### Solution to Problem

Thus, herein, a block copolymer represented by Formulas (I) and (II) below are provided as subject 1.

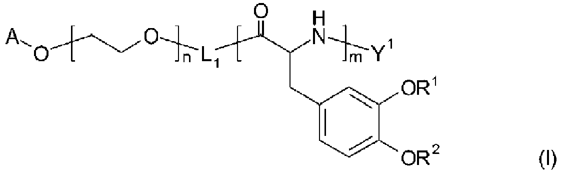

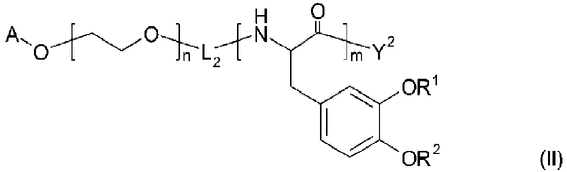

In the formulas above,
A represents an unsubstituted or substituted C₁-C₁₂ alkyl, and when substituted, the substituent in the substituted C₁-C₁₂ alkyl represents a formyl group, or a group represented by Formula R'R"CH-, where R' and R" each independently represent a C₁-C₄ alkoxy, or R' and R" together represent -OCH₂CH₂O-, -O(CH₂)₃O-, or -O(CH₂)₄O-.

L₁ and L₂ each independently represent a linking group.

R¹ and R² each independently represent a protecting group.

Y¹ represents a hydrogen atom; a C₁-C₂₀-alkylcarbonyl that may be substituted by a halogen, a C₁-C₆-alkyloxy, a substituted phenyl, or a perfluoro group; or a benzoyl group. Y² represents a hydroxyl; a C₁-C₂₀-alkyloxy that may be substituted by a halogen, a C₁-C₆-alkyloxy, a substituted phenyl, or a perfluoro group; or a benzyl group.

m represents an integer from 2 to 100, and n represents an integer from 4 to 1000.

In the present specification, more specifically, the following subjects, and the subjects related to use thereof are also provided.
Subject 2: the copolymer of subject 1, wherein the linking group L₁ represents a single bond, -(CH₂)ₐ-NH-, -(CH₂)ₐ-O-, -S-, or -Ph- (phenylene), and the linking group L₂ represents a carbonyl, -(CH₂)ₐ-C(O)-, -C(O)-(CH₂)ₐ-C(O)-, -PhC(O)-, or - Ph-NH-, where "a" is an integer of 1 to 6, and the protecting groups R¹ and R² each independently represent a C₁-C₆-alkylcarbonyl that may be substituted by a halogen, a C₁-C₆-alkyloxy, or a substituted phenyl.
Subject 3: the copolymer according to subject 1, which is represented by Formula (I).
Subject 4: a nanomicelle containing the copolymer according to any of subjects 1 to 3.
Subject 5: a formulation for prevention or treatment of Parkinson's disease, the formulation containing as an active ingredient the copolymer according to any of subjects 1 to 3 or the nanomicelle of subject 4.
Subject 6: the formulation for prevention or treatment of Parkinson's disease according to subject 5, wherein the formulation contains as an additional active ingredient at least one selected from an L-DOPA decarboxylase inhibitor, a monoamine oxidase B (MAO-B) inhibitor, a catechol-O-methyltransferase (COMT) inhibitor, a noradrenaline enhancers, an adenosine A_{2A} receptor stimulant, an L-DOPA and/or dopamine antagonist, and a dopamine D2 receptor blocker.
Subject 7: the copolymer according to any of subjects 1 to 3 for use in prevention or treatment of Parkinson's disease.
Subject 8: the nanomicelle according to subject 4 for use in prevention or treatment of Parkinson's disease.
Subject 9: a method of preventing or treating Parkinson's disease, the method including administering a prophylactically or therapeutically effective amount of the copolymer according to subject 1 to a subject in need of the administration.
Subject 10: a method of preventing or treating Parkinson's disease, the method including administering a prophylactically or therapeutically effective amount of the nanomicelle of subject 4 of subject 1 to a subject in need of the administration.

### Effects of Invention

The block copolymers represented by Formulas (I) and (II) exhibit high retention in blood. On the other hand, enzymes in vivo in warm-blooded animals can eliminate the protecting groups at the 3,4-positions on the 3,4-dihydroxy-L-phenylalanine unit and can gradually cleave the peptide bonds of the polymer backbone. This can gradually release free L-DOPA into the blood over a long time, thus effectively relieves symptoms characteristic of Parkinson's disease and allows the suppression of levodopa-induced dyskinesia (LID).

### Brief Description of Drawings

FIG. 1 is a conceptual diagram schematically illustrating a design of a nanomedicine using a L-DOPA-containing block copolymer and a process after in vivo administration.
FIG. 2 are ¹HNMR spectra of L-DOPA(OAc)₂ and L-DOPA(OAc)₂-NCA.
FIG. 3 is a ¹HNMR spectrum of a PEG-b-P(L-DOPA(OAc)₂).
FIG. 4 is a diagram showing a DLS measurement result of a micellar solution of a PEG-b-P(L-DOPA(OAc)₂) in water.
FIG. 5 is a graph showing pH stability test results of micellar nanoparticles of a PEG-b-P(L-DOPA(OAc)₂) in water using DLS.
FIG. 6 is a diagram showing results of a disintegration test of a micelle by enzymatic degradation of a PEG-b-P(L-DOPA(OAc)₂) in aqueous solutions.
FIG. 7 is a graph showing results of a cytotoxicity test.
FIG. 8 is a schematic diagram of a verification schedule for therapeutic effect of micellar nanoparticles of a PEG-b-P(L-DOPA(OAc)₂) on Parkinson's disease in Test 2 (1).
FIG. 9 is a graph showing results of a grid walking test.
FIG. 10 is a graph showing measurement results of a resting tremor test.
FIG. 11 is a graph showing measurement results of a narrow beam walk test.
FIG. 12 is a graph showing measurement results of body weight change in the verification of therapeutic effect of micellar nanoparticles of a PEG-b-P(L-DOPA(OAc)₂) on Parkinson's disease.
FIG. 13 is a schematic diagram of a verification schedule for suppressive effect of the nanoparticles on L-DOPA-induced dyskinesia in Test 2 (5).
FIG. 14 is a graph showing measurement results of AIMS.
FIG. 15 is a graph showing measurement results of a slider test.
FIG. 16 is photographs of stained pictures of various organs (heart, lung, liver, spleen, and kidney) related to Nano^{DOPA} administration to Parkinson's disease model mice.
FIG. 17 is a graph showing changes in the concentration of L-DOPA in the blood by intraperitoneal administration of drugs.
FIG. 18 is a graph showing changes in the concentration of L-DOPA in the blood by oral administration of drugs.
FIG. 19 is a graph showing AUCs of L-DOPA in the blood by oral administration of drugs.
FIG. 20 is a schematic diagram of a treatment schedule for mouse models in Test 6.
FIG. 21 is a graph showing effects on Parkinson's disease mouse models by oral administration of drugs

### Detailed Description of Invention

The aspects or subject matter disclosed in the present specification will be described below. All abbreviations and technical terms described are used to represent the meaning or contents commonly used in the art or commonly understood by those skilled in the art unless otherwise indicated. Unless the context clearly indicates otherwise, the word "or" includes "and". When used, the words "comprising", "comprise", "containing", or the like mean "including". Thus, for example, "comprising A or B" means "comprising A" or "comprising B" or "comprising A and B" and in addition means that any other constituent features other than A or B may also be included.

### [1] Block copolymer

Each term or each group defining the block copolymer has the following meanings, and the following specific examples can be given.

The linking group means a single bond or a divalent linking group linking the PEG segment and the 3,4-protected L-DOPA segment, and is composed of up to 34, preferably up to 18, or more preferably up to 10 atomic groups, a C₁-C₆-alkyl, -CO-, - O-, -NH-, -NR^{r}- (where "r" can be a C₁-C₆-alkyl, a C₁-C₆-alkyloxy, or the like), a phenylene group, or the like. L₁ can be, but not limited to, a single bond, -(CH₂)ₐ-NH-, -(CH₂)ₐ-O-, or -S-, and L₂ is represented by a carbonyl, -(CH₂)ₐ-C(O)-, -C(O)-(CH₂)ₐ-C(O)-, -PhC(O)-, or -Ph-NH-, where "a" is an integer of 1 to 6. The directionalities of these groups are each assumed to correspond to any of the chemical structure formulas in Formulas I and II.

In both formulas, "m" can be an integer from 2 to 100, from 2 to 50, from 2 to 20, or from 2 to 10, and "n" can be an integer from 4 to 1000, from 8 to 1000, or from 10 to 500.

The protecting group means a group that bonds to a hydroxyl, a reactive functional group present in 3,4-positions of L-DOPA, masks or reduces its reactivity, and can be hydrolyzed in the bodies of warm-blooded animals or subjects including humans (which may hereinafter also be referred to as "in vivo"). In other words, the protecting group can be exemplified by protecting groups used to form prodrugs of L-DOPA and can be, for example, but not limited to, a C₁-C₆-alkylcarbonyl that may be substituted by a halogen, a C₁-C₆-alkyloxy, or a substituted phenyl, or benzoyl. Specific examples of these groups include acetyl, pivaloyl, benzylcarbonyl, and benzoyl. These groups can be identical or different but are preferably identical for the object of the present invention, and acetyl is preferably selected. The present inventors propose a method using a 1 M acetic acid solution of hydrogen chloride in place of gaseous hydrogen chloride as a means to avoid the risks associated with the method in the art to protect the hydroxyls. Throughout the present specification, an expression such as C₁-C₆- or C₁-C₂₀- means a disclosed group having from 1 to 6 or from 1 to 12 carbon atoms.

The block copolymers represented by Formulas I and II, in which the hydrophilic chain polyethylene glycol (PEG) segment and the segment having the hydrophobized polyamino acids are covalently bonded via a linking group, exhibit amphiphilicity. Thus, in a polar solvent, for example, water, these segments are presumed to form a micelle by hydrophobic interaction, the micelle composed of the PEG segment, which is highly mobile in water, as a shell and the hydrophobized polyamino acid segment as the core, and thus can be understood to improve the retention in blood. It is understood that although such a micellar structure is formed, the block copolymers represented by Formulas I and II forming this micellar structure undergo the enzyme actions in vivo, thus the protecting groups are eliminated and the polymer backbones are cleaved, and ultimately free L-DOPA is released and exhibits the intrinsic physiological activity. Measurement results of dynamic light scattering (DLS) revealed that such a micelle has an average particle size of 20 to 80 nm in an aqueous medium or water. The micelle forming operation itself can be performed according to a known method.

Such copolymers can be manufactured by separately preparing the (PEG) segment and the hydrophobized polyamino acid segment and linking them to form the linking group described above. The former can be provided by ring-opening anionic polymerization of ethylene oxide, a method known per se, using an initiator corresponding to the A moiety. The latter can be provided by solution- or solid-phase peptide synthesis, known per se, using 3,4-hydroxy-protected L-DOPA. However, the copolymer of Formula (I) can also be provided by ring-opening anionic polymerization of an N-carboxylic anhydride (NCA) of 3,4-hydroxy-protected L-DOPA using a macroinitiator containing the PEG segment although a specific example will be disclosed later.

### (2) Use

The block copolymer itself or its micelle of the above aspects or subject matter can be used as an active ingredient to prevent or treat Parkinson's disease. An embodiment of such use can be a formulation or composition for prevention or treatment of Parkinson's disease, the formulation or composition containing the copolymer or micelle as an active ingredient, and can be the copolymer or micelle used to prevent or treat Parkinson's disease, and still can also be a method of preventing or treating Parkinson's disease, the method including administering a prophylactically or therapeutically effective amount of the copolymer or micelle to a subject (human or warm-blooded animal) in need of the administration.

Such a copolymer, especially a micelle, is stable under a wide range of conditions including low pH conditions except under extreme basic conditions and thus can be administered to subjects (including warm-blooded animals besides humans) including patients via any route of administration, oral or parenteral. Such a formulation or a composition can contain a pharmaceutically acceptable carrier, or a pharmaceutically acceptable diluent or excipient in addition to the block copolymer as an active ingredient. Properties of the carrier, diluent, or excipient depend on a specific administration form used. For a solid composition including those for oral administration (e.g., in a form of powders, pills, tablets, or capsules), such an excipient can include nontoxic excipients commonly used in the art, for example, an excipient, such as mannitol, lactose, and lactose; disintegrating agents, such as starch; lubricants, such as magnesium stearate; and binders, such as hydroxylpropyl cellulose. For parenteral administration, the block copolymer itself can be solubilized in an aqueous medium (such as water, a buffered distilled water, and a buffered saline), and thus the carrier, diluent, or excipient can be such a medium (such as water, saline, a buffered distilled water, or a balanced salt solution), glucose, mannitol, lactose, aqueous dextrose, glycerose, or the like. The form of the parenteral formulation or composition can be provided as a solution, suspension, or the like.

Furthermore, such a formulation or composition can contain an active ingredient other than the block copolymer as long as the active ingredient does not adversely affect the effect of the intended use of the disclosed formulation. Examples of such an active ingredient can include, but not limited to, L-DOPA decarboxylase inhibitors, monoamine oxidase B (MAO-B) inhibitors, catechol-O-methyltransferase (COMT) inhibitors, noradrenaline enhancers, adenosine A_{2A} receptor stimulants, L-DOPA and/or dopamine antagonists, and dopamine D2 receptor blockers. Examples of each active ingredient are not limited to the following, but the examples for L-DOPA decarboxylase inhibitors can include carbidopa and benserazide, examples for MAO-B inhibitors can include selegiline, rasagiline, and zonisamide, examples of COMT inhibitors can include entacapone, examples for noradrenaline enhancer can include droxidopa, examples for adenosine A_{2A} receptor stimulants can include amantadine, pramipexole, ropinirole, rotigotine, apomorphine, cabergoline, bromocriptine, percolide, and Azilect, examples for L-DOPA and/or dopamine antagonists can include istradefylline, and examples of dopamine D2 receptor blockers can include trihexyphenidyl, promethazine, biperiden, profenamine, Mexan, and piroheptine.

Such a formulation or composition can be a mixed formulation or in a form of a combination of each individual formulation. These formulations or compositions can each be administered orally or parenterally (e.g., as an injection, intravenously, subcutaneously, enterally, or intraperitoneally) to a subject at from 0.1 to 1000 mg per day, preferably at from 200 mg to 750 mg in terms of the block copolymer (L-dopa).

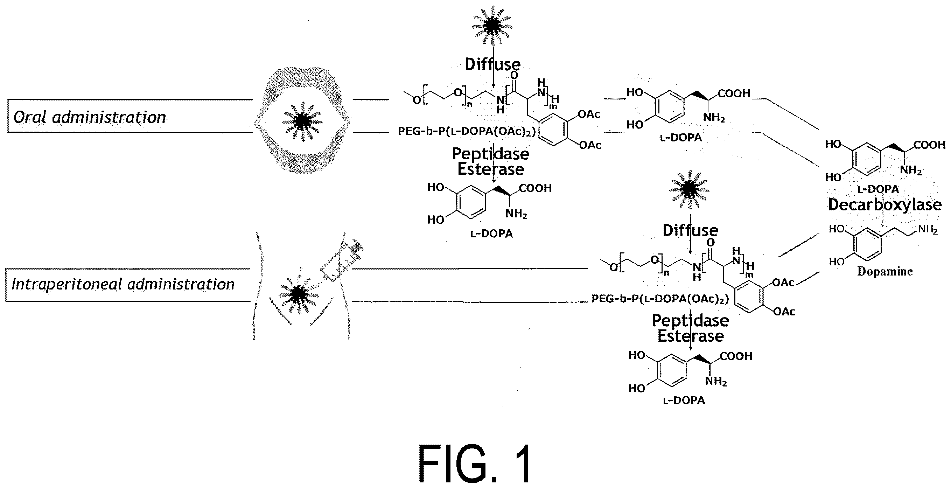

To facilitate the understanding of the above description, a conceptual diagram illustrating the copolymer and a transition of a drug after administration is provided as FIG. 1.

Parkinson's disease is a disorder caused by the lack of neurotransmitter dopamine in the brain, a neurotransmitter involved in movement. Movement disorders include representative symptoms, such as resting tremor, muscle rigidity (muscular rigidity), bradykinesia, and postural reflex, which are called the four major symptoms.

### Examples

The above subjects will be described below with reference to specific examples, but here, to avoid complexity, a block copolymer belonging to the block copolymer represented by Formula (Ia) in which the protecting groups are acetyls will be described as examples, but the scope of the present invention is not limited to these examples.

### Manufacturing Example 1: synthesis of copolymer

The block copolymer according to subject 1 can be obtained based on the synthetic route illustrated in Scheme 1 below.

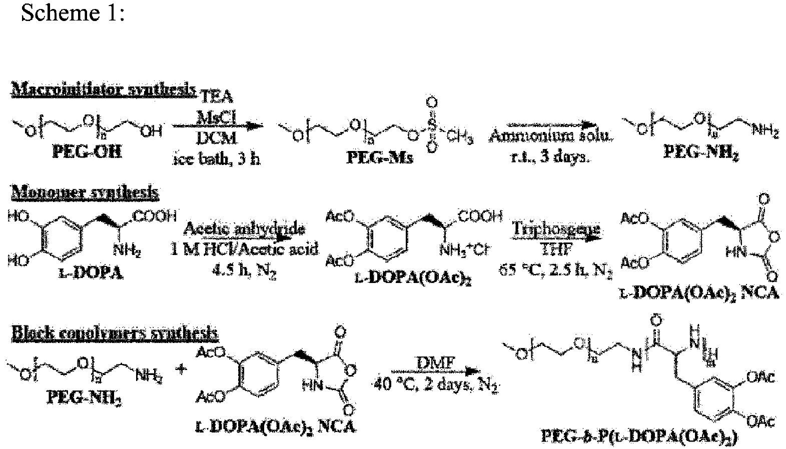

As seen in the scheme above, the synthesis is broadly divided into the synthesis of a PEG-NH₂ (PEG is an abbreviation for a poly(ethylene glycol) or poly(oxyethylene)) serving as a macroinitiator, the synthesis of an L-DOPA(OAc)₂-NCA used as a monomer, and the synthesis of a PEG-b-P(L-DOPA(OAc)₂) using the PEG-NH₂ and the L-DOPA(OAc)₂-NCA and extending the L-DOPA(OAc)₂ chain from the PEG end.

### (1) Macroinitiator synthesis (syntheses of PEG-Ms and PEG-NH₂)

In 60 mL of dichloromethane (DCM), 10 g (2 mmol) of MeO-PEG-OH was dissolved. To this solution, 2.8 mL (20 mmol) of triethylamine (TEA) and 0.8 mL (10 mmol) of methanesulfonyl chloride were added, and the mixture was allowed to react under ice cooling for 4 hours. The reaction solution was purified by precipitation with isopropyl alcohol (IPA), and a white powder was obtained by drying under reduced pressure (a yield of 9.41 g, a yield ratio of 93.2%, and an introduction ratio of 100%).

In 70 mL of 28% ammonia water, 9 g of PEG-Ms was dissolved and allowed to react at room temperature for 3 days. The reaction solution was purified by reprecipitation with IPA, and a white powder was obtained by drying under reduced pressure (a yield of 8.60 g, a yield ratio of 96.6%, and an introduction ratio of 100%).

The formation of PEG-Ms and PEG-NH₂ was confirmed by ¹HNMR measurements.

### (2) Monomer synthesis (syntheses of L-DOPA(OAc)₂ and L-DOPA(OAc)₂-NCA)

A flask containing 5 g of L-DOPA was purged with nitrogen, 100 mL of 1 M HCI/CH₃COOH (AOS) was further added under a nitrogen atmosphere, and the mixture was stirred at room temperature for 2 hours. To this solution, 5 mL of acetic anhydride was added under a nitrogen atmosphere and allowed to react at room temperature for 1.5 hours, then 5 mL of acetic anhydride was added again and allowed to react at 54°C for 1 hour. After evaporating and suffieicntly concentrating the reaction solution, 15 mL of ethanol was added to quench unreacted materials, and 3,4-diacetyloxylevodopa (L-DOPA(OAc)₂) formed was purified by precipitation with ethyl ether. A white powder was obtained by drying under reduced pressure (a yield of 4.34 g, a yield ratio of 60.9%, and an introduction ratio or 95.5%).

In 150 mL of tetrahydrofuran (THF), 3.17 g (10 mmol) of L-DOPA(OAc)₂ and 1.48 g (5 mmol) of triphosgene were dissolved, and the mixture was allowed to react at 65°C for 2.5 hours. After the reaction solution was reprecipitated in hexane, the precipitate containing N-carboxylic anhydride (NCA) of L-DOPA(OAc)₂ was dissolved in 50 mL of THF. The solution was added to 500 mL of hexane with stirring, and L-DOPA(OAc)₂-NCA was reprecipitated. The reprecipitation operation was performed twice, and then a white powder of L-DOPA(OAc)₂-NCA was obtained by drying under reduced pressure (a yield of 2.85 g, a yield ratio of 92.8%, and a reaction ratio of 93.0%).

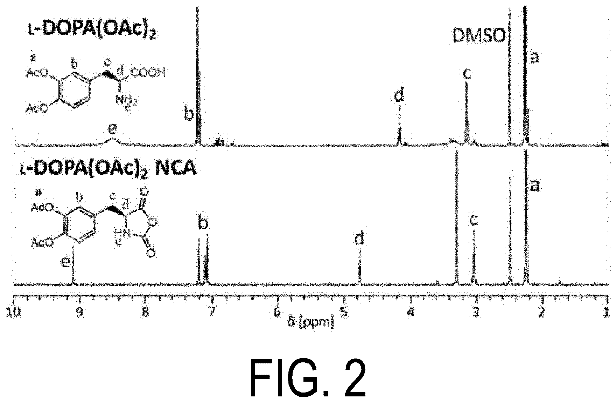

¹HNMR spectra of L-DOPA(OAc)₂ and L-DOPA(OAc)₂-NCA are shown in FIG. 2.

### (3) Block copolymer synthesis (synthesis of PEG-b-P(L-DOPA(OAc)₂))

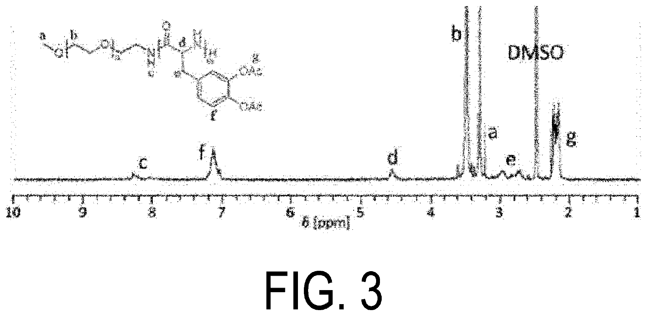

After 1.25 g (2.5 mmol) of PEG-NH₂ was dissolved in 2 mL of benzene and the solution was lyophilized, 12.5 mL of dimethylformamide (DMF) was added and dissolved under a nitrogen atmosphere. In a flask, 1.54 g (5 mmol) of L-DOPA(OAc)₂-NCA was weighed out, and the flask was purged with nitrogen. The dimethylformamide (DMF) solution of PEG-NH₂ described above was added under a nitrogen atmosphere, and the mixture was allowed to react at 40°C for 2 days. The reaction solution was purified by reprecipitation with diethyl ether, and a white powder was obtained by drying under reduced pressure (a yield of 1.68 g, a yield ratio of 60.1%, and the number of polyamino acid units of 8.03). A ¹HNMR spectrum of the block copolymer is shown in FIG. 3.

### Manufacturing Example 2: preparation of micelle

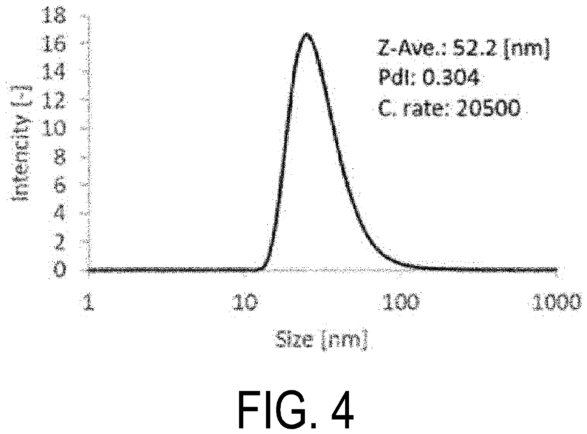

In 5 mL of DMF, 0.5 g (in terms of L-DOPA) of the PEG-b-P(L-DOPA(OAc)₂) was dissolved, and the solution was added dropwise to 5 mL of distilled water with stirring. This was dialyzed overnight against distilled water using a dialysis membrane (fractional molecular weight (MWCO): 3500 Da), then diluted with distilled water to a volume of 400 mL, and a 1.25 mg/mL micellar solution was prepared. Finally, 3.6 g of sodium chloride was added to make 0.9% saline. A DLS measurement result of such a micellar solution is shown in FIG. 4.

### Test 1: in vitro test

### (1) Stability of micellar aqueous solution of PEG-b-P(L-DOPA(OAc)₂) under pH variation

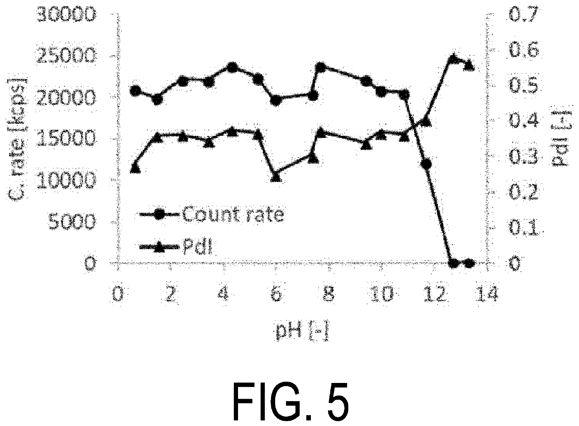

To examine the stability of the copolymer when the pH of the solution was varied, DLS measurements were performed on the copolymer in the micellar state in the solution. The results are shown in FIG. 5. As apparent from FIG. 5, the micelle can be stably present even under acidic conditions and thus can be administered orally (however, the copolymer is deprotected under extreme basic conditions).

### (2) Degradation behavior of PEG-b-P(L-DOPA(OAc)₂) in micellar aqueous solution by peptidase

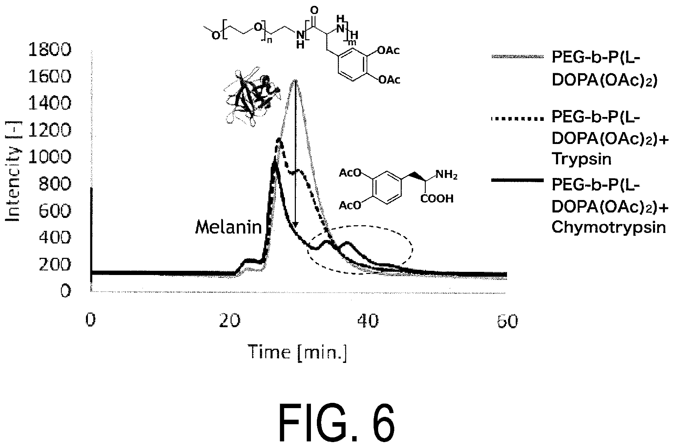

Micelle disintegration tests were performed using chymotrypsin, trypsin, or esterase as enzymes. The results are shown in FIG. 6. From FIG. 6, it can be understood that peptide bonds of the backbone are degraded (but cannot be degraded by trypsin) and the acetyl groups are deprotected by esterase.

### (3) Toxicity assessment test of PEG-b-P(L-DOPA(OAc)₂) micelle by MTT assay using normal cells (BAEC)

Briefly, this test was performed as follows. Cultured BAEC cells were seeded in a 96-well plate, a sample solution was added, and the cells were incubated for 24 hours. The MTT reagent was then added, and the cells were incubated for 4 hours. To visualize formazan produced by the metabolism of living cells, an SDS-HCl solution was then added, and the mixture was allowed to react for additional 4 hours. Finally, cell activity was examined by measuring absorbance at 570 nm using a microplate.

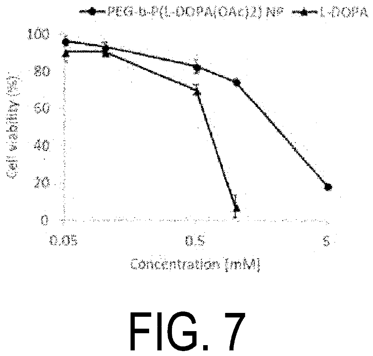

The test results are shown in FIG. 7. FIG. 7 revealed improved retention in blood and decreased cytotoxicity.

### Test 2: in vivo tests

A mouse model of Parkinson's disease was created.

Male C57BL/6J mice, 15 weeks old, were used (see Chonpathompikunlert, P. (2018). BMC Complementary and Alternative Medicine, 18(103), 1-12). A method of intraperitoneally administering 1-methyl-4-phenyl -1,2,3,6-tetrahydropyridine (MPTP) was used, but MPTP was administered before and after treatment with intraperitoneal administration of nanoparticles because of high recovery ability of mice themselves. The administration before treatment was to induce PD, and the administration after treatment was to visualize by a behavioral test.

### (1) Therapeutic effect of the PEG-b-P(L-DOPA(OAc)₂) micelle (or nanoparticles) on Parkinson's disease was verified under the following conditions.

The mice were randomly divided into the following four groups (n = 4).
1) Saline, for 12 days
2) MPTP 20 mg/kg, 4 times daily, every 2 hours
   + saline, for 11 days + MPTP 30 mg/kg, for 3 days
3) MPTP 20 mg/kg, 4 times daily, every 2 hours
   + L-DOPA 15 mg/kg, for 11 days + MPTP 30 mg/kg, for 3 days
4) MPTP 20 mg/kg, 4 times daily, every 2 hours
   + NPs 15 mg/kg (in terms of L-DOPA), for 11 days + MPTP 30 mg/kg, for 3 days

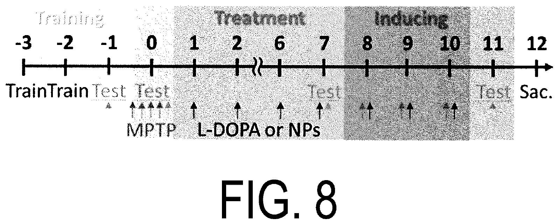

The verification schedule for the therapeutic effect of the nanoparticles on Parkinson's disease is shown in FIG. 8.

### (2) Grid walking test

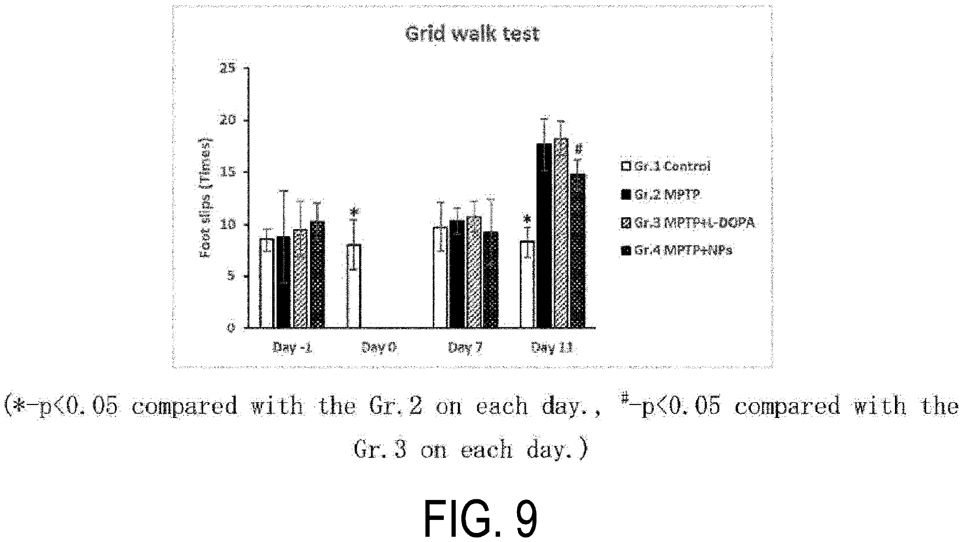

A grid walk test was performed as a behavioral test according to the above schedule, and the number of times the limbs of the mouse slipped into the opening was counted to test the balance ability. Mice with a strong symptom of Parkinson's disease usually have a large number of slipping, but on Day 0, because it was immediately after MPTP administration, the mice had a severe symptom and thus had difficulty in walking except for the mice in the control group (not administered with MPTP), and this resulted in a value of zero. On Day 7, the behavioral test showed some recovery, and no difference could be observed. Thereafter, MPTP was administered again, and on Day 11, no difference was observed in the L-DOPA-administered group compared to the non-treatment group, but the number of slipping significantly decreased in the nanoparticles (NPs)-administered group compared to the L-DOPA-administered group. This confirmed that L-DOPA is a small molecule and thus was rapidly metabolized and failed to exert sufficient effect, whereas the nanoparticles exerted a superior therapeutic effect on Parkinson's disease with improved retention in the blood. See FIG. 9, which shows the test results.

### (3) Resting tremor test

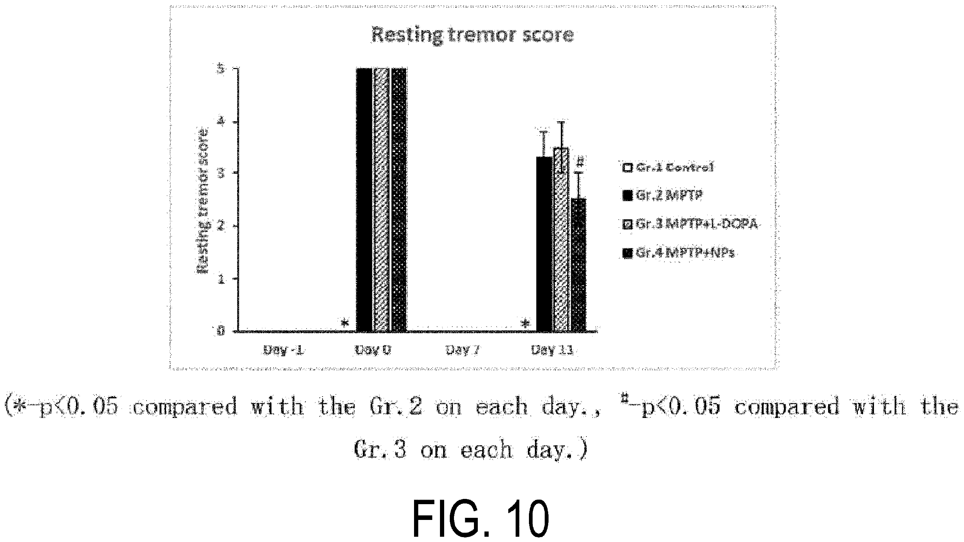

In this test, the severity of resting tremor, a symptom characteristic of Parkinson's disease, was scored (0: no tremor, 1: slight muscle tremor, 2: occasional tremor to the head, 3: apparent tremor but not always involving tremor to the head, 4: the limbs and head cannot be moved because of continuous tremor, 5: continuous and severe tremor of the whole body). The results are shown in FIG. 10.

In FIG. 10, mice with a strong symptom have larger scores. On Day 11, the previous therapeutic effect was persistently exerted only in the NPs-administered group.

The PEG-b-P(L-DOPA(OAc)₂)-NPs that are produced by the present invention and can sustainably release L-DOPA in response to enzymes in the body can prolong the therapeutic effect on Parkinson's disease, further suppress levodopa-induced dyskinesia, and can be greatly expected as a new therapeutic drug for Parkinson's disease.

### (4) Narrow beam walk test

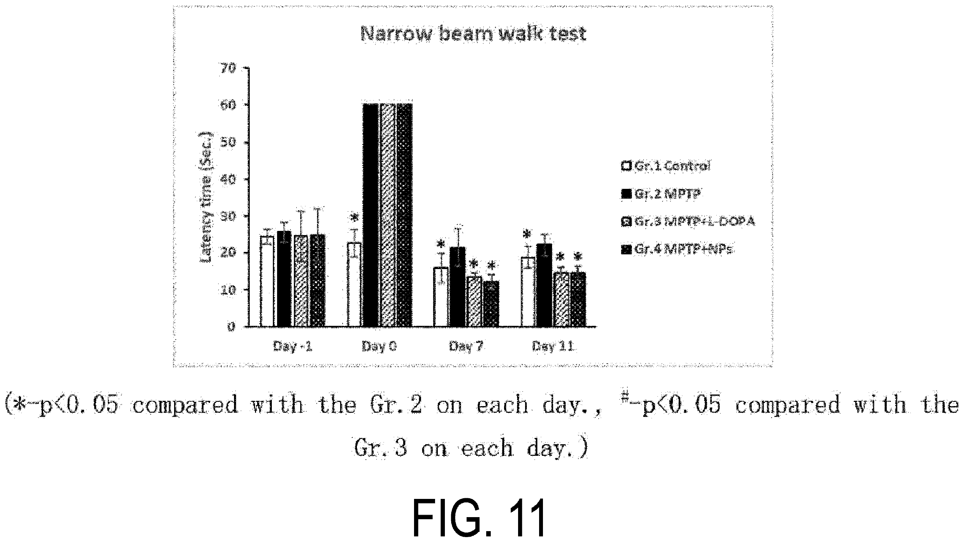

In this test, motor ability can be accessed by measuring the time until a mouse passes through a narrow bridge with a width of 1 cm, and the time increases with Parkinson's disease. The results are shown in FIG. 11. As apparent from FIG. 11, in this test, decrease in motor ability and improvement in therapeutic effect were continuously and significantly observed in the L-DOPA and NP groups over the MPTP-administered group also on Day 7, and similar trends were also observed on Day 11 after MPTP was administered again.

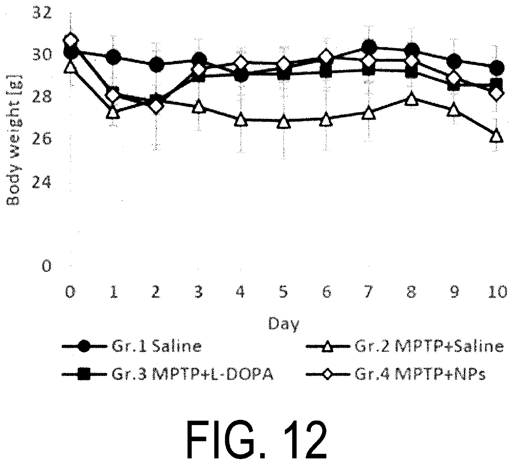

In addition, although body weight loss due to MPTP was observed in this experiment, the body weight rapidly recovered in the L-DOPA-administered group and the NPs-administered group, and no noticeable health damage was observed during the administration. See FIG. 12.

### (5) Verification test of suppressive effect of PEG-b-P(L-DOPA(OAc)₂) micelle (or nanoparticles) on L-DOPA-induced dyskinesia

The mice were randomly divided into the following four groups (n = 4).
1) Saline, for 18 days
2) MPTP 20 mg/kg, 4 times daily, every 2 hours
   + saline, for 17 days + MPTP 30 mg/kg, for 3 days
3) MPTP 20 mg/kg, 4 times daily, every 2 hours
   + L-DOPA 25 mg/kg, Benserazide 12 mg/kg, for 17 days
   + MPTP 30 mg/kg, for 3 days
4) MPTP 20 mg/kg, 4 times daily, every 2 hours
   + NPs (in terms of L-DOPA) 25 mg/kg, Benserazide 12 mg/kg, for 17 days
   + MPTP 30 mg/kg, for 3 days

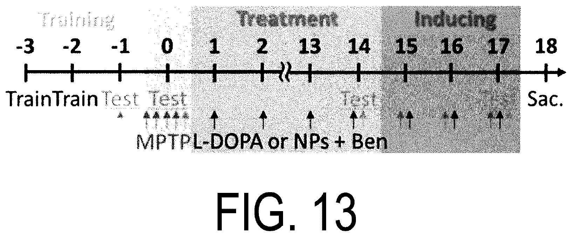

The verification schedule for the suppressive effect of the nanoparticles on L-DOPA-induced dyskinesia is shown in FIG. 13.

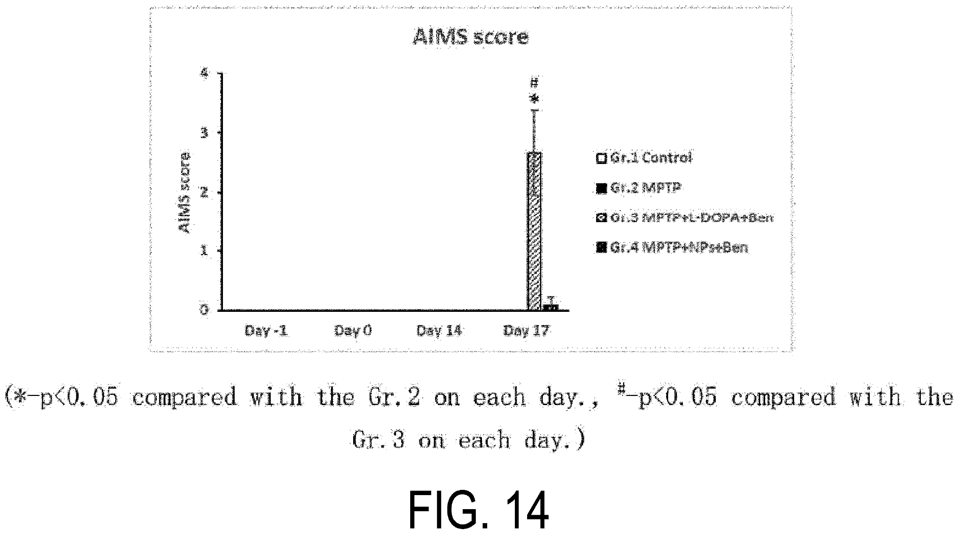

An abnormal involuntary movement scale test (AIMS test) was performed as a behavioral test according to the above schedule, and the severity of dyskinesia was scored (0: no AIM, 1: AIM in <50%, 2: AIM in > 50%, 3: continuous but stopped by stimulation, 4: continuous). The test results are shown in FIG. 14. Mice with a strong symptom of dyskinesia have a large score. On Day 17, involuntary movements of the oral cavity and twisting of the body axis characteristic of dyskinesia were observed only in the L-DOPA-administered group, and only some weakness of the limbs was observed in the MPs-administered group. This demonstrates that the use of nanoparticles according to the present invention allows maintaining a constant concentration of L-DOPA in the body and suppressing levodopa-induced dyskinesia.

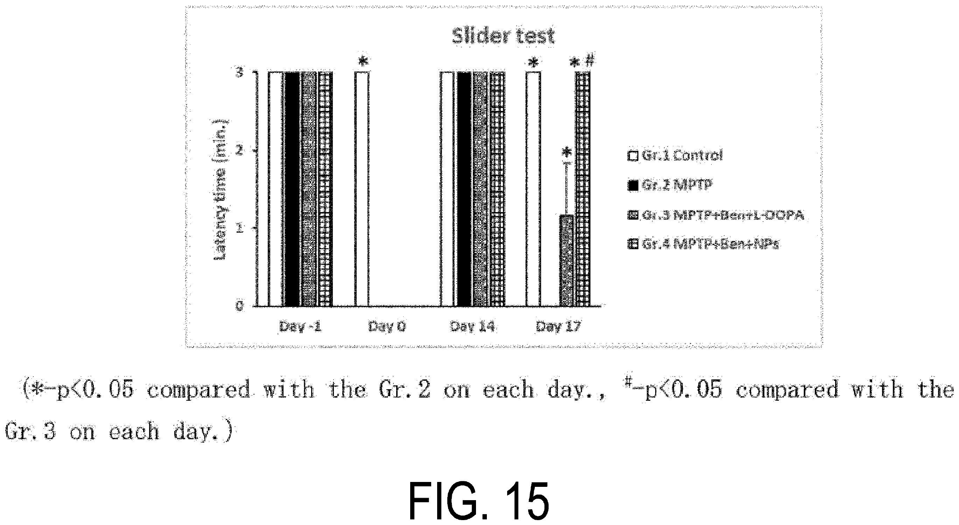

(6) In addition, in a slider test, motor ability can be assessed by measuring the time until each mouse becomes unable to walk on a slope of 26.5 degrees or slides down, and the time is expected to be shortened with Parkinson's disease. After MPTP administration, the mice had difficulty in walking due to weakness, and on Day 17, sliding down due to involuntary movements of mice was observed only in the L-DOPA-administered group, whereas mice were able to continue to walk in the NPs-administered group. See FIG. 15.

### Test 3: Histological analysis

After the animals were euthanized, major organs were cut out from mouse models of Parkinson's disease (PD) and L-DOPA-induced dyskinesia (LID) and fixed in a 4% paraformaldehyde solution at 4°C for 72 hours. These tissues were then processed using an automated tissue processor (TP1020, Leica, Germany). The samples embedded in paraffin were subsequently sliced to a thickness of 5 mm with an automatic microtome and stained with hematoxylin and eosin (H&E) as well as Masson trichrome (MT). Histological evaluation was performed by observation under an optical microscope (DP73, Olympus, Japan) and assessment of the potential toxicity of the PEG-b-P(L-DOPA(OAc)₂)-Npa (or Nano^{DOPA}) during the treatment.

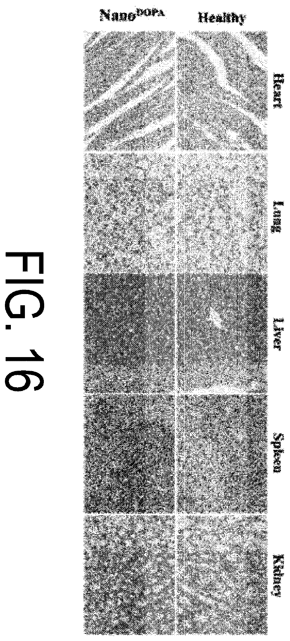

The results of the H&E staining are illustrated in FIG. 16. In the figures, the upper row illustrates photographs of the stained samples from a normal mouse and the lower row illustrates photographs of the stained samples from a mouse in the Nano^{DOPA}-administered group. These figures clearly demonstrate that administration of Nano^{DOPA} to the Parkinson's disease model mice does not damage each organ at all.

### Test 4: Pharmacokinetic study

L-DOPA (25 mg/kg; 127 mmol/kg) or Nano^{DOPA} (105 mg polymer/kg; 127 mmol L-DOPA/kg) was injected intraperitoneally into ICR mice. The mice were euthanized after a predetermined time, and plasma was collected. L-DOPA in plasma was extracted using cold phosphate-buffered saline (PBS (pH 7.4)), then sedimented by centrifugation at 15000 rpm for 15 minutes, and a sample filtered using a 0.2-µm membrane was stored at -80°C until analysis. The amounts of L-DOPA in plasma and tissue were measured using an LC-MS/MS system (API 2000, AB SCIEX, Canada) under the following conditions in a column (TSKgel ODS-100Z)⁽¹⁾.
Note ⁽¹⁾: See Sintov, A. C., Levy, H. V., & Greenberg, I. (2017). Continuous transdermal delivery of L-DOPA based on a self-assembling nanomicellar system. Pharmaceutical Research, 34(7), 1459-1468.
Eluent: a mixture of 0.5% acetic acid in water and 0.5% acetic acid in acetonitrile (a volume ratio of 95:5)
Flow rate: 0.2 mL/min

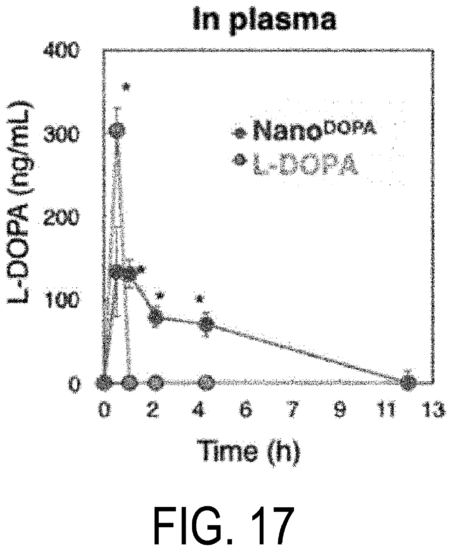

The results (changes in the concentration of L-DOPA in the blood by intraperitoneal administration) are shown in FIG. 17. As apparent from FIG. 17, after the intraperitoneal administration, the concentration of L-DOPA in the blood rapidly increases initially, and L-DOPA cannot be detected after 1 hour, whereas the rapid increase in the blood concentration is suppressed by the intraperitoneal administration of Nano^{DOPA}, and a constant concentration in the blood is maintained over a long time.

### Test 5: Uptake of Nano^{DOPA} to blood by oral administration

The concentrations in the blood were measured in a manner similar to Test 4, except that the L-DOPA and Nano^{DOPA} solutions each were administered orally (administered with a tube) instead of i.p. administration. The dose of L-DOPA was 25 mg/kg (127 mmol/kg), and the dose of Nano^{DOPA} was 105 mg polymer/kg (127 mmol L-DOPA/kg).

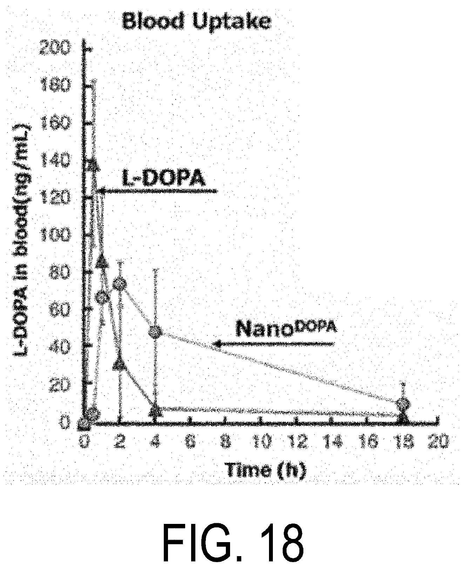

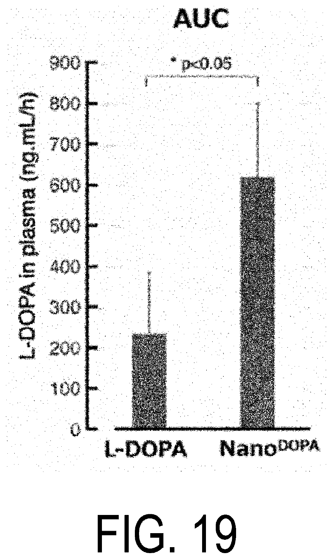

The results (changes in the concentration of L-DOPA in the blood by oral administration) are shown in FIG. 18, and the area under the curves (AUCs) of L-DOPA by oral administration are shown in FIG. 19.

As seen in FIG. 18, the concentration of L-DOPA in the blood rapidly increased initially and rapidly metabolized in the oral administration, whereas the rapid increase in the blood concentration was suppressed by the oral administration of Nano^{DOPA}, and the concentration in the blood was maintained over a long time, and the AUC increased twice or more as shown in FIG. 19.

### Test 6: Verification of effect of Nano^{DOPA} by oral administration on Parkinson's disease mouse models

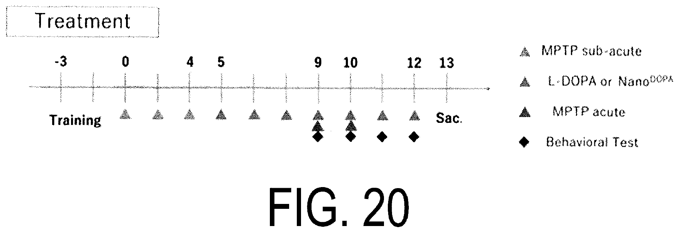

Male C57BL/6J mice, 15 weeks old, were used as models as in Test 2. A three-day training period was provided for the models prior to administration to familiarize them with the experimental apparatus. MPTP was administered intraperitoneally at 30 mg/kg every day for the first 5 days (sub-acute). After the administration of MPTP, oral administration of L-DOPA or Nano^{DOPA} (administered once daily with a tube) was started, and MPTP was administered intraperitoneally to Groups II and III on Day 9, 4 times daily, every 2 hours (acute). MPTP was also administered intraperitoneally in a similar manner to the remaining Group IV on Day 10. The catalepsy test was performed on the same day as the acute administration of MPTP. In addition, the narrow beam walk test (see Test 2 (4)) was performed 2 days after the catalepsy test. All the mice were euthanized the next day of the behavioral test. A schematic diagram of the treatment schedule is shown in FIG. 20.

Each group described above was as follows, and the mice were randomized into the following four groups (n = 8).
I. Control
II. MPTP 30 mg/kg, for 5 days (sub-acute) + MPTP 20 mg/kg, 4 times daily, every 2 hours (acute)
III. MPTP 30 mg/kg, for 5 days (sub-acute) + MPTP 20 mg/kg, 4 times daily, every 2 hours (acute) + L-DOPA 15 mg/kg (oral)
IV. MPTP 30 mg/kg, for 5 days (sub-acute) + MPTP 20 mg/kg, 4 times daily, every 2 hours (acute) + Nano^{DOPA} 15 mg/kg (given ad libitum)

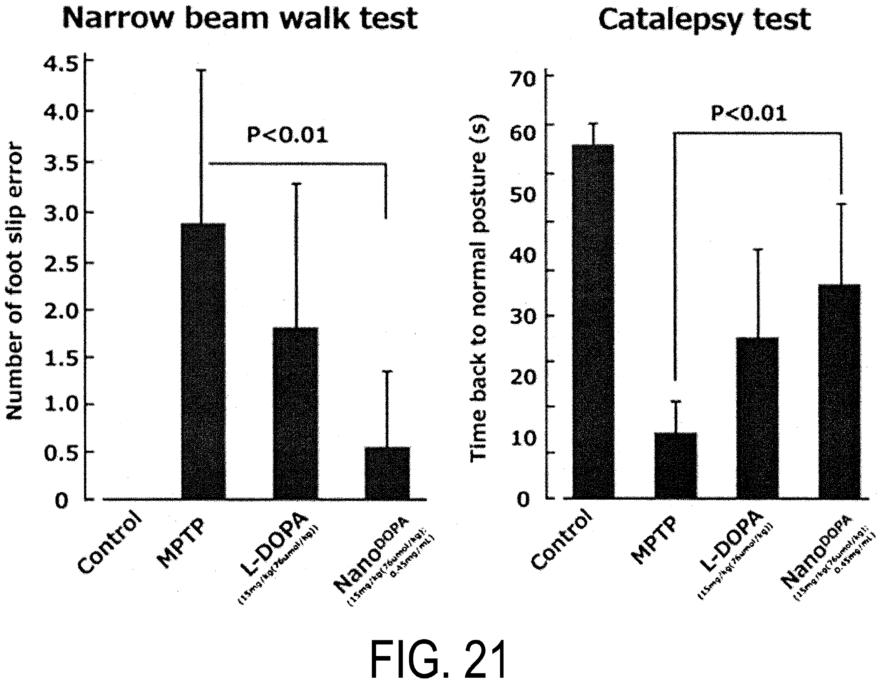

The results are shown in FIG. 21. The figure on the left shows the results of the narrow beam walk test (gait disturbance on the narrow bar is estimated by the number of foot slip errors), and the figure on the right shows the results of the catalepsy test (the ability to maintain a posture passively forced to take and not to try to change the posture at own will).

The MPTP-administered group showed increased foot slip erros in the narrow beam walk test, whereas the L-DOPA group significantly suppressed the same. In addition, the catalepsy test revealed significant reduction in the MPTP-administered group and no significant difference in L-DOPA but significant increase in the Nano^{DOPA} group, demonstrating the effectiveness of the oral administration.

The above test results reveals that the PEG-b-P(L-DOPA(OAc)₂)-NPs produced by the present invention can sustainably release L-DOPA in response to enzymes in the body, thus can prolong the therapeutic effect on Parkinson's disease, and can further suppress L-DOPA-induced dyskinesia. Thus, the PEG-b-P(L-DOPA(OAc)₂)-NPs can be greatly expected as a new therapeutic drug for Parkinson's disease.

### Industrial Applicability

As described above, the block copolymer disclosed in the present specification prolongs the therapeutic effect on Parkinson's disease and can further suppress L-DOPA-induced dyskinesia. Thus, the block copolymer can be utilized in the pharmaceutical industry.

## Claims

1. A block copolymer represented by Formulas (I) and (II), 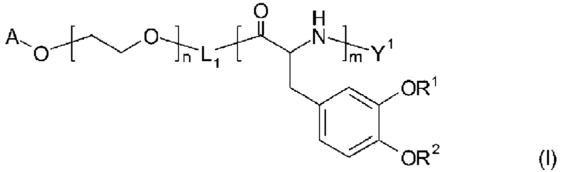 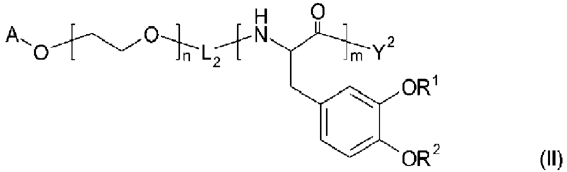 wherein each variable abbreviation is as follows:
A represents an unsubstituted or substituted C₁-C₁₂ alkyl, and when substituted, a substituent in the substituted C₁-C₁₂ alkyl represents a formyl group, or a group represented by Formula R'R"CH-, where R' and R" each independently represent a C₁-C₄ alkoxy, or R' and R" together represent -OCH₂CH₂O-, -O(CH₂)₃O-, or -O(CH₂)₄O-;
L₁ and L₂ each independently represents a linking group;
R¹ and R² each independently represents a protecting group;
Y¹ represents a hydrogen atom; a C₁-C₂₀-alkylcarbonyl that may be substituted by a halogen, a C₁-C₆-alkyloxy, a substituted phenyl, or a perfluoro group; or a benzoyl group; Y² represents a hydroxyl; a C₁-C₂₀-alkyloxy that may be substituted by a halogen, a C₁-C₆-alkoxy, a substituted phenyl, or a perfluoro group; or a benzyloxy group;
m represents an integer from 2 to 100; and
n represents an integer from 4 to 1000.

2. The copolymer according to claim 1,
wherein the linking group L₁ represents a single bond, -(CH₂)a-NH-, -(CH₂)a-O-, -S-, or -Ph-, and the linking group L₂ represents a carbonyl, -(CH₂)a-C(O)-, - C(O)-(CH₂)a-C(O)-, -PhC(O)-, or -Ph-NH-, where a is an integer of 1 to 6, and
wherein the protecting groups R¹ and R² each independently represent a C₁-C₆-alkylcarbonyl that may be substituted by a halogen, a C₁-C₆-alkyloxy, or a substituted phenyl.

3. The copolymer according to claim 1 or 2, which is represented by Formula (I).

4. A nanomicelle comprising the copolymer claimed in any one of claims 1 to 3.

5. A formulation for prevention or treatment of Parkinson's disease, the formulation comprising as an active ingredient the copolymer claimed in any one of claims 1 to 3 or the nanomicelle described in claim 4.

6. The formulation for prevention or treatment of Parkinson's disease according to claim 5, wherein the formulation comprises as an additional active ingredient at least one selected from: an L-DOPA decarboxylase inhibitor, a monoamine oxidase B (MAO-B) inhibitor, a catechol-O-methyltransferase (COMT) inhibitor, a noradrenaline enhancer, an adenosine A_{2A} receptor stimulant, an L-DOPA and/or dopamine antagonist, and a dopamine D2 receptor blocker.

7. The copolymer according to any one of claims 1 to 3 for use in prevention or treatment of Parkinson's disease.

8. The nanomicelle according to claim 4 for use in prevention or treatment of Parkinson's disease.

9. A method of preventing or treating Parkinson's disease, the method comprising administering a prophylactically or therapeutically effective amount of the copolymer claimed in claim 1 to a subject in need of the administration.

10. A method of preventing or treating Parkinson's disease, the method comprising administering a prophylactically or therapeutically effective amount of the nanomicelle claimedin claim 4 to a subject in need of the administration.
